# EUROPEAN PATENT APPLICATION

(11) **EP 1 163 930 A2**
(43) Date of publication of application: **19.12.2001**
(21) Application number: 01870129.2
(22) Date of filing: 13.06.2001
(51) Int. Cl.: A61N 5/06

(54) **Phototherapy device**

(30) Priority: 13.06.2000 BE 200000379
(71) Applicant: Rainbow-Flash c.v.b.a., 2018 Antwerpen (BE)
(72) Inventor: Vermeire, Leona, 2018 Antwerpen (BE)
(74) Representative: Luys, Marie-José A.H.

(57) **Abstract**

A phototherapy device for emitting a selectable spectrum of light towards a person comprising first, respectively second light paths (10, 20) for transmitting first, respectively second light beams (11, 21), first, respectively second filtering means (12, 22) for selecting first, respectively second spectra from the first, respectively second light beams (11, 21), and mixing means (2) for mixing the first and second light beams (11, 21) to a third light beam (31) which is directed towards the person, the mixing means (2) being provided to generate the third light beam (31) by passing a first percentage of the first light beam (11) and reflecting a second percentage of the second light beam (21) in the same direction.

## Description

The present invention relates to a phototherapy device for emitting a selectable spectrum of light towards a person, according to the preamble of the first claim.

It has long been recognised that light can have profound psychological as well as physiological effects on the human organism. Although both ocular as well as non-ocular techniques have been employed in an attempt to achieve various such effects, ocular treatment appears to be most efficacious. Not only are the eyes highly specialised organs specifically adapted for sensing light, but a sizeable portion of the brain is exclusively devoted to processing data generated by the retinas. Moreover, neurologists and anatomists have relatively recently demonstrated the existence of nerve pathways extending from the retinas that are separate and apart from the pathways linked to the sight centre of the brain, more particularly the hypothalamus, the epiphyses and the hypophyses. These newly discovered interconnections link the eyes with neurological centres in the brain that influence and control many of the body's regulatory functions. Such regulatory centres typically exert their control via neurological or biochemical means.

It has been suggested that phototherapy may effectively be employed to influence the function of organs to thereby influence sleep cycles, feeding cycles, reproduction cycles and other biological rhythms, which may have been disrupted by for example stress, shift work or jet lag. Additionally, it has been found that some of the body's responses to light are acutely dependent upon specific characteristics of the light perceived by the eyes such as the light's wavelength and intensity. During a phototherapy session, particular responses of the treated person's body can be elicited or enhanced by varying such characteristics according to certain sequences or patterns.

A phototherapy device is for example known from US-A-5,046,494. The device described in US-A-5,046,494 comprises two light sources for emitting light into two separate parallel light paths. A filter wheel having a plurality of different colour filters is rotatably mounted at the end of each light path, by means of which a limited spectrum of light can be filtered from the light emitted by the light source. By rotating the wheel, a different colour filter can be brought into the light path for selecting a different spectrum. Beyond the filter wheels, the two parallel light paths converge into a single light path, in which a common diffuser is mounted for mixing and diffusing the light coming from both light paths. Finally, an aperture wheel having a plurality of apertures of different sizes is rotatably mounted in the common light path. By means of this aperture wheel the amount of light emitted into the person's eyes can be selected. So by means of the two light paths which each have different colour filter sets, a wide variety of colours can be selected for the light emitted towards the person.

The phototherapy device known from US-A-5,046,494 has the disadvantage that the light emitted towards the person is insufficiently uniform in colour.

It is an object of the invention to provide a phototherapy device in which the light of two light paths can be mixed to a substantially uniform colour.

This object is achieved according to the invention with a phototherapy device showing the technical features of the characterising part of the first claim.

The phototherapy device of the invention comprises a first light path for emitting a first light beam and a second light path for emitting a second light beam, both light beams being directed towards the mixing means for mixing the first and second light beams to the third light beam which is directed towards the person. The mixing means are provided to generate the third light beam by, on the one hand, passing a first percentage of the first light beam and, on the second hand, reflecting a second percentage of the second light beam. The passed first percentage of the first light beam and the reflected second percentage of the second light beam are emitted by the mixing means in the same direction. In other words the passed first percentage of the first light beam coincides with the reflected second percentage of the second light beam.

In the prior art phototherapy device, the two light paths emit two parallel light beams onto the diffuser, so that the third beam which is obtained may comprise light which changes gradually from the first colour of the first light path on one side to the second colour of the second light path on the other side. So in the prior art, the third beam does not have a uniform colour when different colours are selected in the two light paths. In the phototherapy device of the invention, the mixing of light is achieved by coinciding light beams, so that a third beam may be obtained which is substantially uniform in colour, even when the first and second filter means are selected such that they filter different colours from the first and second beams.

Furthermore, as the mixing means are provided to mix light from the first and second light beams in a direct way, i.e. without mixing the light by diffusing it in a diffuser, a third light beam may be obtained which has a higher intensity with respect to the prior art.

The first percentage is preferably substantially equal to the second percentage, so that both the first and second spectra selected from the first and second light beams are substantially equally represented in the spectrum of the third light beam which is directed towards the person. However, the first percentage may also differ from the second percentage, so that either the first or the second light beam is more prominent in the third light beam.

In a preferred embodiment of the phototherapy device of the invention, the first and second light paths are provided to emit the first and second light beams in intersecting directions. This embodiment of the device further comprises a semi-transparent mirror, which is located at a position where the first and second light beams intersect and is directed according to the bisector plane of the first and second light beams. The semi-transparent mirror reflects 50% and passes 50% of the incoming light in both directions. As the mirror is directed according to the bisector plane, the angles of incidence of both light beams on the mirror are substantially equal. As the mirror is located where the light beams intersect, the light beams coincide on the opposite sides of the mirror. In this way, a third light beam is obtained by transmission of 50% of the first light beam through the mirror and reflection of 50% of the second light beam on the mirror. This third beam is in line with the first light beam and is directed towards the person.

As the mirror is semi-transparent, it passes 50% of the second light beam and reflects 50% of the first light beam, which light is mixed into a fourth light beam in line with the second light beam. This has the advantage that the fourth light beam has substantially the same spectral composition and intensity as the third light beam. As a result, it can for example be directed towards a side screen on the exterior of the device for use as a reference for selecting the spectral composition and the intensity of the light emitted towards the person.

The first and second beams are preferably emitted by the first and second light paths in substantially perpendicular directions, the angles of incidence of the light beams on the mirror being approximately 45°. The directions in which the first and second light beams are emitted may however also enclose angles narrower or wider than 90°.

Preferably, the light paths are each provided with a light conductor extending between the light source and the filter means. The light conductors in both light paths are preferably of substantially equal length and are preferably both connected to a common light source. In this way, both light paths are provided to transmit light of the same spectral composition and intensity and it may also be obtained that the light travelling distance between the light source and the mirror is substantially equal for both light paths. This has the advantage that the phototherapy device of the invention is suitable for working with a light source which emits light pulses, since a light pulse created by the common light source reaches the mirror via both light paths on substantially the same moment. In this way, a substantially uniform mixture of two colours of light pulses can be achieved.

The light source used in the phototherapy device of the invention is preferably a halogen lamp which is provided with means for varying the electric current which is fed to the lamp or the electric tension over the lamp. In this way, the lamp can be controlled for emitting continuous light or pulsing light which varies in intensity, such as for example a cyclic light wave with light of alternatively increasing and decreasing intensity, or a continuation of light pulses. The light source may however also be an incandescent lamp or any other light source known to the person skilled in the art.

The filter means which are mounted in each light path preferably each comprise a filter wheel having a plurality of colour filters. These colour filters are preferably narrow-band filters with a bandwidth of for example about 10 nm. In this way, a very narrow spectrum of light, i.e. a very clear colour can be selected in each light path, so that, after mixture, the light beam emitted towards the person may also have a very narrow bandwidth and a very clear colour.

As the phototherapy device of the invention allows the person to be irradiated with intense light of a substantially uniform colour and having a narrow bandwidth, the device can reduce the amount of phototherapy needed for acquiring healing effects in the person.

The phototherapy device of the invention will be further elucidated by means of the following description and the appended figures.

Figure 1 shows a schematic view of a preferred embodiment of the phototherapy device according to the invention.

Figure 2 shows a filter wheel mountable in each light path of the phototherapy device according to the invention.

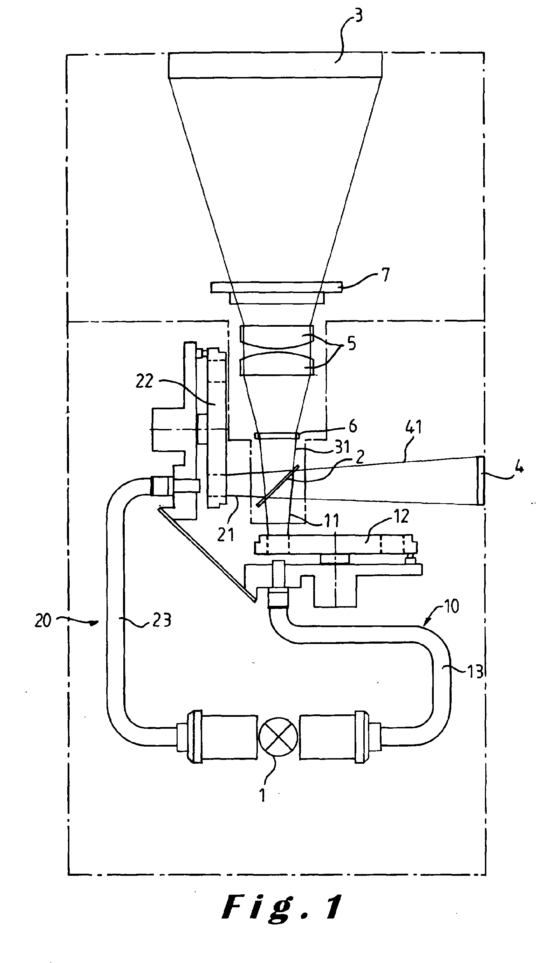

The phototherapy device shown in figure 1 comprises a first light path 10 for transmitting a first light beam 11 and a second light path 20 for transmitting a second light beam 21. The first and second light beams 11, 21 are generated by a light source 1 which is common to both light paths 10, 20. Each light path is provided with filter means 12, 22 for selecting first and second spectra from the first, respectively second light beams 11, 21. The first and second light paths 10, 20 are constructed such that the first light path 10 emits the first light beam 11 in a direction substantially perpendicular to and intersecting with the second light beam 21 emitted by the second light path 20. Where the first and second light beams 11, 21 intersect, a semi-transparent mirror 2 is mounted for mixing the first and second light beams 11, 21. The mirror 2 is directed according to the bisector plane of the first and second light beams 11, 21.

The semi-transparent mirror 2 passes 50% of the incoming light on both sides and reflects the other 50% of the incoming light. In this way, the mirror 2 is provided to mix the first and second light beams 11, 21, which coincide on opposing areas of the mirror 2, to third and fourth light beams 31, 41. The third light beam 31 is in line with the first light beam 11 and results from the 50% of the first light beam 11 which is passed by the mirror 2 and the 50% of the second light beam 21 which is reflected on the mirror 2. The fourth light beam 41 is in line with the second light beam 21 and results from the 50% of the second light beam 21 which is passed by the mirror 2 and the 50% of the first light beam 11 which is reflected on the mirror 2. As a result, the third as well as the fourth light beam 31, 41 are composed of 50% of the first and second light beams 11, 21 and have substantially the same colour and intensity. The third light beam 31 is directed towards a front screen 3 and is used for irradiating the eyes of a person, whereas the fourth light beam 41 is directed towards a side screen 4 and is used as a reference for controlling the colour and intensity of the third light beam 31.

In the embodiment shown in figure 1, the first light beam 11 is emitted in a direction substantially perpendicular to the second light beam 21. The directions in which the first and second light beams 11, 21 are emitted may however also enclose an angle which is wider or narrower than 90°. In any case, the mirror 2 is directed according to the bisector plane of the directions in which the first and second light beams 11, 21 are emitted.

The mirror 2 is preferably semi-transparent so that 50% of the incoming light is reflected on both sides of the mirror and 50% is passed. This has the advantage that the third and fourth light beams 31, 41 are of substantially the same spectral composition and intensity. The mirror 2 may however also have a different degree of transparency.

Between the mirror 2 and the front screen 3, a number of optical devices 5, 6, 7 are mounted for modifying the third light beam 31. These optical devices may comprise the following:
- one or more lenses 5 for converging or diverging the light beam,
- a glider opening 6 for inserting a glider (not shown) having an aperture with a particular shape, for obtaining a third light beam having said particular shape (for example a circle, a star, a triangle or any other shape known to the person skilled in the art),
- and a polarisation filter 7 for obtaining an equally polarised third light beam.
The optical devices may however also comprise any other optical device known to the person skilled in the art.

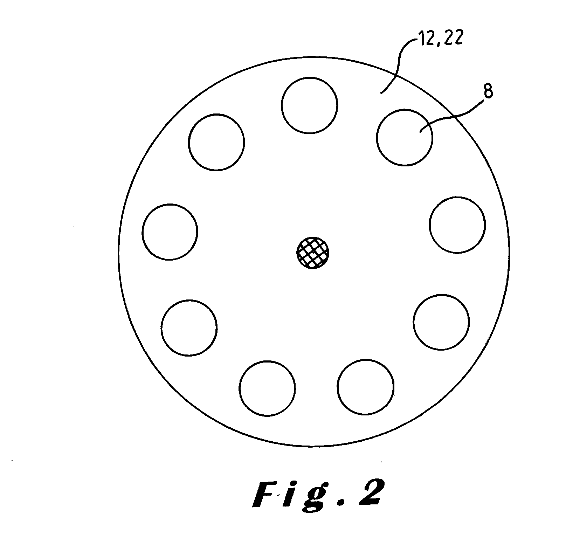

The filter means 12, 22 in the first and second light paths 10, 20 preferably comprise a filter wheel 12, 22 (cf. figure 2). These filter wheels 12, 22 each comprise a number of different colour filters 8, which are equally divided along the periphery of the wheels 12, 22. Each colour filter 8 is preferably a narrow-band filter having a bandwidth of about 10 nm, for filtering 10 nm from the light spectrum emitted by the light source 1. Preferably, the light emitted by the light source 1 comprises the whole of the visible light spectrum (380-780 nm), so that any colour can be filtered from its light, but the light source may also be part of the daylight spectrum. The wheels 12, 22 are rotatably mounted in their respective light paths 10, 20 and are provided with rotation means (not shown). By operating these rotation means, a different colour filter 8 can be brought into the light path 10, 20.

Each light path 10, 20 comprises a light conductor 13, 23 for transmitting the first and second light beams 11, 21 from the light source 1 towards the mirror 2. The light conductors 13 and 23 are preferably of substantially equal length, so that the distance which the light travels through the first path 10 is substantially equal to the distance which the light travels through the second path 20.

The common light source 1 is preferably a halogen lamp 1 which is provided with means (not shown) for varying the electric current which is fed to the lamp or the electric tension over the lamp. These means make it possible to control the light emitted by the halogen lamp 1. In this way, a selection can be made between continuous light and pulsing light which varies in intensity, such as for example a cyclic light wave with light of alternatively increasing and decreasing intensity, or a continuation of light pulses. These means also allow the pulsing frequency of the light pulses or the light wave to be varied. The light source 1 may however also be an incandescent lamp, daylight or any other light source known to the person skilled in the art.

The device is preferably operable by means of electronic operating means (not shown), for controlling the colour of the first and second light beams and for controlling the light emitted by the light source. The electronic operating means may be integrated into the device or be incorporated in a separate control box which is connected to the device by means of an electronic cable.

## Claims

1. A phototherapy device for emitting a selectable spectrum of light towards a person comprising first, respectively second light paths (10, 20) for transmitting first, respectively second light beams (11, 21), first, respectively second filtering means (12, 22) for selecting first, respectively second spectra from the first, respectively second light beams (11, 21), and mixing means (2) for mixing the first and second light beams (11, 21) to a third light beam (31) which is directed towards the person, **characterised in that** the mixing means (2) are provided to generate the third light beam (31) by passing a first percentage of the first light beam (11) and reflecting a second percentage of the second light beam (21) in the same direction.

2. A phototherapy device according to claim 1, **characterised in that** the first light path (10) is provided to emit the first light beam (11) in a first direction and the second light path (20) is provided to emit the second light beam (21) in a second direction, the first direction enclosing an angle and intersecting with the second direction, and that the mixing means (2) comprise a semi-transparent mirror (2) located at a position where the first and second directions intersect and directed according to the bisector plane of the first and second directions.

3. A phototherapy device according to claim 2, **characterised in that** the semi-transparent mirror (2) is provided to mix the first and second light beams (11, 21) into a fourth light beam (41) of substantially the same spectral composition and intensity as the third light beam (31), the fourth light beam (41) being directed in a direction different from the direction of the third light beam and being used as a reference of the spectral composition and intensity of the third light beam.

4. A phototherapy device according to claim 2 or 3, **characterised in that** the first direction is substantially perpendicular to the second direction.

5. A phototherapy device according to any one of claims 1-4, **characterised in that** the first, respectively second light paths (10, 20) are provided with first, respectively second light conductors (13, 23), the first and second light conductors (13, 23) being of substantially equal length and being connected to a common light source (1).

6. A phototherapy device according to any one of claims 1-5, **characterised in that** the light source (1) is a halogen lamp which is provided with means for varying the electric current which is fed to the lamp or the electric tension over the lamp.

7. A phototherapy device according to any one of claims 1-6, **characterised in that** the first and second filter means (12, 22) each comprise a filter wheel (12, 22) having a plurality of different colour filters (8) equally divided along its periphery.

8. A phototherapy device according to claim 7, **characterised in that** each colour filter (8) has a bandwidth of about 10 nm.

9. A phototherapy device according to any one of claims 1-8, **characterised in that** the device further comprises a polarisation filter (7) for polarising the light of the third light beam (31).
